# FASCICULE DE BREVET EUROPEEN

(11) **EP 1 140 951 B1**
(45) Date de publication et mention de la délivrance du brevet: **03.05.2006**
(21) Numéro de dépôt: 99961130.4
(22) Date de dépôt: 21.12.1999
(51) Int. Cl.: C07F 9/50, B01J 31/24

(54) **PREPARATION DE COMPOSES ORGANOPHOSPHORES SULFONES HYDROSOLUBLES**
HERSTELLUNG VON WASSERLÖSLICHEN SULFONIERTEN ORGANOPHOSPHOR-VERBINDUNGEN
METHOD FOR PREPARING WATER-SOLUBLE SULPHONATED ORGANOPHOSPHOROUS COMPOUNDS

(30) Priorité: 23.12.1998 FR 9816586
(43) Date de publication de la demande: 10.10.2001
(73) Titulaire: RHODIA POLYAMIDE INTERMEDIATES, 69192 Saint-Fons (FR)
(72) Inventeur: AOUNI, Larbi, F-69500 Bron (FR); BURATTIN, Paolo, F-69002 Lyon (FR); COQUERET, Pierre, F-69340 Francheville (FR); HUSER, Marc, F-69100 Villeurbanne (FR)
(74) Mandataire: Esson, Jean-Pierre
(86) Numéro de dépôt international: PCT/FR1999/003234
(87) Numéro de publication internationale: WO 2000/039134

(56) Documents cités:
- EP-A- 0 133 410
- EP-A- 0 175 919

## Description

La présente invention concerne un procédé de préparation de composés organophosphorés sulfonés hydrosolubles, notamment convenable pour les réactions organiques réalisées avec une catalyse biphasique.

Les composés organophosphorés trouvent une application particulièrement importante comme ligands d'éléments métalliques de transition pour réaliser des systèmes catalytiques de différentes réactions organiques telles que la carbonylation, l'hydroformylation, l'hydrocyanation, l'isomérisation de composés oléfiniques.

Ces systèmes catalytiques sont utilisés généralement dans des réactions mettant en oeuvre une seule phase et donc requiert une étape quelquefois complexe de séparation et récupération du catalyseur.

Dans le début des années 1970, il a été proposé des composés hydrosolubles pouvant former des complexes avec des éléments métalliques à l'état d'oxydation zéro. Ces composés hydrosolubles appartiennent généralement à la famille des composés organophosphorés comprenant au moins un groupement sulfonate. Ainsi, F. JOES et M.T. BECK dans un article publié dans React. Kim. Catal. Letters 2(1975) 257 et BAWOSKI et al dans un article publié dans la revue Nouv. J. Chem. 2(1978) 137 décrivent une triphénylphosphine monosulfonée soluble dans l'eau et non extractible par des solvants organiques.

La synthèse de ces produits a permis le développement d'un nouveau mode de catalyse appelé la catalyse biphasique. En effet, le catalyseur, constitué par des éléments métalliques de transition complexés par les composés organophosphorés hydrosolubles est présent dans une phase aqueuse tandis que les réactifs sont dans une phase organique. L'agitation du milieu et sa mise en émulsion permet d'obtenir une catalyse efficace. En fin de réaction, le catalyseur est récupéré par simple séparation par décantation des deux phases.

La société RHONE-POULENC a développé cette technique pour la réalisation de plusieurs réactions organiques importantes comme l'hydroformylation d'oléfines pour la production d'aldéhydes comme décrit dans les brevets français 2 505 322 et 2 541 675. Une autre application importante de ce système catalytique, décrit notamment dans les brevets français n° 2 338 253 et 2 366 237 concerne la réaction d'hydrocyanation d'oléfines et l'isomérisation des nitriles obtenus pour par exemple la synthèse de l'adiponitrile qui est un grand intermédiaire chimique notamment pour la fabrication des monomères du polyamide.

Des travaux sont constamment entrepris pour améliorer l'économie de ces procédés, notamment en augmentant la durée de cycle et durée de vie du système catalytique, ainsi que la diminution de la consommation de catalyseur par quantité d'adiponitrile produit.

Au cours de ces travaux, il a été constaté une dégradation des composés organophosphorés sulfonés hydrosolubles.

Un des buts de la présente invention est notamment de remédier à ces inconvénients en proposant un ligand organophosphoré sulfoné hydrosoluble résistant à la dégradation, et donc proposer un système catalytique biphasique plus stable.

A cet effet, l'invention concerne un procédé de mise en oeuvre de réactions organiques par catalyse biphasique comprenant une phase organique contenant les réactifs et les produits de la réaction, une phase aqueuse comprenant un catalyseur et un composé organophosphoré sulfoné hydrosoluble caractérisé en ce que le composé organophosphoré sulfoné hydrosoluble a subi une élimination des composés ou radicaux sulfites.

Selon une caractéristique préférentielle de l'invention, le composé organophosphoré sulfoné présente une concentration pondérale en sulfite inférieure à 100 ppm, de préférence inférieure à 50 ppm.

Selon une autre caractéristique de l'invention, l'élimination des sulfites est obtenue par transformation de ceux-ci en gaz sulfureux.

Parmi les réactions organiques catalysées par une catalyse biphasique on peut citer les réactions d'hydroformylation, carbonylation, d'oxydation, d'isomérisation, d'hydrocyanation, notamment appliquées à des composés insaturés.

Selon un mode de réalisation préféré, on citera notamment la réaction d'hydrocyanation des composés organiques comprenant au moins une liaison éthylénique permettant de fabriquer des composés nitriles tels que l'adiponitrile pour la synthèse notamment, des monomères amines, aminoacides ou lactames des polyamides. Ce procédé de synthèse comprend notamment une hydrocyanation du pentène-3 nitrile en adiponitrile, pour laquelle le catalyseur comprenant comme composant une organophosphine désulfitée conformément à l'invention est particulièrement convenable.

Ainsi, dans le cas d'une telle application la durée de vie du catalyseur peut être fortement améliorée. Cette amélioration est constatée, dans le cas de l'hydrocyanation de composés organiques comprenant plusieurs liaisons éthyléniques et notamment des diènes, aussi bien pour la réaction d'hydrocyanation de la première liaison éthylénique que dans celle de la seconde liaison éthylénique.

Le procédé de l'invention permet de réaliser l'hydrocyanation du butadiène pour la synthèse de l'adiponitrile avec un catalyseur à base de ligands organophosphorés sulfonés hydrosolubles et d'au moins un élément métallique à l'état d'oxydation zéro, avec une consommation de catalyseur par kg d'adiponitrile produit nettement plus faible que celle constatée avec un catalyseur ou un ligand n'ayant pas subi d'élimination des sulfites.

Selon un mode de réalisation préféré de l'invention et cela est un autre objet de l'invention, l'élimination des sulfites contenus dans le composé organophosphoré sulfoné hydrosoluble est obtenue par abaissement du pH de la solution de composés organophosphorés à une valeur inférieure ou égale à 4 et maintien de la solution à un pH inférieur ou égal à 4 jusqu'à l'obtention d'une concentration en sulfite dans la solution inférieure à 100 ppm.

Le dosage des composés sulfites dans la solution est réalisé, par exemple, par chromatographie ionique.

L'abaissement du pH de la solution peut être réalisé par tout moyen approprié. Toutefois, selon un mode de réalisation préféré de l'invention, cet abaissement est obtenu par addition d'un acide fort minéral ou organique sous forme pure ou plus préférentiellement de solution.

La solution acide peut être une solution concentrée ou diluée.

Comme acide convenable pour l'invention, on peut citer les acides présentant par exemple un pKa inférieur ou égal à 4, les anhydrides correspondants et plus généralement tout composé inerte chimiquement vis à vis du composé oraganophosphoré et pouvant abaisser le Ph d'une solution.

A titre d'exemple non limitatif, on peut citer comme acides l'acide sulfurique, l'acide chlorhydrique, l'acide trifluoroacétique, l'acide paratoluène sulfonique, l'acide perchlorique, l'acide nitrique.

Par ailleurs, la solution de composés organophosphorés sulfonés hydrosolubles est de préférence une solution aqueuse. Toutefois, des solutions utilisant comme solvant des mélanges eau/alcool sont également convenables. L'alcool peut être remplacé par tout solvant miscible à l'eau.

Selon une autre caractéristique de l'invention, la solution est maintenue à un pH inférieur ou égal à 4, à une température inférieure à 100°C avantageusement comprise entre 40°C et 90°C.

Le gaz sulfureux produit par la transformation des sulfites est, dans un mode de réalisation préféré de l'invention, éliminé du milieu par entraînement par un fluide vecteur. Ce fluide vecteur est de préférence non oxydant. Ainsi, les fluides vecteurs convenables pour l'invention sont, par exemple, l'azote, le gaz carbonique, la vapeur d'eau, les gaz rares ou inertes, de l'air appauvri en oxygène.

Les composés organophosphorés sulfonés hydrosolubles convenables pour être traités selon le procédé de l'invention et utiles comme ligands dans les procédés de catalyse biphasique, sont généralement les composés organophosphorés sulfonés préparés en mettant en oeuvre une ou plusieurs étapes de sulfonation comme cela est décrit dans l'article paru dans J. Chem. Soc. pages 276 - 288 (1958) ou le brevet anglais n° 1 066 261. Ils peuvent également être préparés par réaction du p. chlorobenzène sulfonate de sodium acide avec la diphénylchlorophosphine comme cela est décrit dans l'article H. Schindibauer, Monatsch. Chem. **96**, pages 2051 - 2057 (1965).

Généralement, ces procédés de synthèse des composés organophosphorés sulfonés ne permettent pas d'obtenir un composé exempt de composés ou radicaux sulfites. En conséquence, pour éviter une dégradation de ces composés au cours de leur utilisation comme catalyseur ou ligand de système catalytique, il est nécessaire selon le procédé de l'invention d'éliminer, au moins partiellement, ces sulfites.

Comme composés phosphines sulfonées hydrosolubles convenables pour mettre en oeuvre l'invention on peut citer les composés appartenant à la famille des composés phosphines décrits dans le brevet français n° 2 338 253 ou dans les demandes de brevet WO 97.12857 et EP 0 650 959.

Ainsi des phosphines convenables pour l'invention répondent à la formule générale (I) suivante : 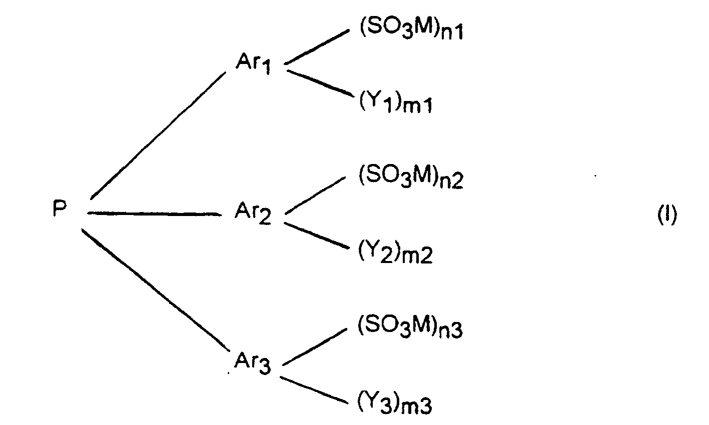 dans laquelle :
* Ar₁, Ar₂ et Ar₃, identiques ou différents, représentent des groupements aryles
* Y₁, Y₂ et Y₃, identiques ou différents, représentent
   . un radical alkyle ayant 1 à 4 atomes de carbone,
   . un radical alcoxy ayant 1 à 4 atomes de carbone,
   . un atome d'halogène,
   . un radical CN,
   . un radical NO₂
   . un radical OH,
   . un radical NR₁R₂, dans la formule duquel R₁ et R₂, identiques ou différents, représentent un radical alkyle ayant 1 à 4 atomes de carbone,
* M est un reste cationique minéral ou organique choisi, de manière à ce que le composé de formule (I) soit soluble dans l'eau, dans le groupe comprenant :
   . H⁺,
   . les cations dérivés des métaux alcalins ou alcalino-terreux,
   . N(R₃R₄R₅R₆)⁺ avec R₃,R₄,R₅ et R₆, identiques ou différents, représentant un radical alkyle ayant 1 à 4 atomes de carbone ou un atome d'hydrogène,
   les autres cations dérivés des métaux dont les sels de l'acide benzènesulfonique sont solubles dans l'eau,
* m₁, m₂ et m₃ sont des nombres entiers, identiques ou différents, de 0 à 5,
* n₁, n₂ et n₃ sont des nombres entiers, identiques ou différents, de 0 à 3, l'un d'entre eux au moins étant égal ou supérieur à 1.

Comme exemples de métaux dont les sels de l'acide benzènesulfonique sont solubles dans l'eau, on peut citer le plomb, le zinc et l'étain.

Par l'expression soluble dans l'eau, on entend de manière générale dans le présent texte un composé soluble à au moins 0,01 g par litre d'eau.

Parmi les phosphines de formule (I), on préfère celles pour lesquelles:
- Ar₁, Ar₂ et Ar₃ sont des groupements phényle,
- Y₁, Y₂ et Y₃ représentent des groupements choisis parmi
   . les radicaux alkyle ayant de 1 à 2 atomes de carbone,
   . les radicaux alkoxy ayant de 1 à 2 atomes de carbone,
- M représente un cation choisi parmi le groupe comprenant
   . H⁺
   . les cations dérivés de Na, K, Ca, Ba
   . NH₄⁺
   . les cations tétraméthylammonium, tétraéthylammonium, tétrapropylammonium, tétrabutylammonium
- m₁, m₂ et m₃ sont des nombres entiers de 0 à 3
- n₁, n₂ et n₃ sont des nombres entiers de 0 à 3, l'un au moins étant également supérieur à 1.

Parmi ces phosphines, les plus particulièrement préférées sont les sels de sodium, de potassium, de calcium, de baryum, d'ammonium, de tétraméthylammonium et de tétraéthylammonium, des mono(sulfophényl) diphényl-phosphine, di(sulfophényl) phényl-phosphine et tri(sulfophényl)-phosphine dans les formules desquelles les groupements SO₃ sont de préférence en position méta.

On peut citer comme autres exemples de phosphines de formule (I) pouvant être mises en oeuvre dans le procédé de l'invention, les sels alcalins ou alcalino-terreux, les sels d'ammonium, les sels d'ammonium quaternaire des (sulfo-3 méthyl-4 phényl) di (méthyl-4 phényl)-phosphine ; (sulfo-3 méthoxy-4 phényl) di(méthoxy-4 phényl)-phosphine ; (sulfo-3 chloro-4 phényl) di(chloro-4 phényl)-phosphine ; di(sulfo-3 phényl) phényl-phosphine ; di(sulfo-4 phényl) phényl-phosphine ; di(sulfo-3 méthyl-4 phényl) (méthyl-4 phényl)-phosphine ; di(sulfo-3 méthoxy-4 phényl) (méthoxy-4 phényl) phosphine; di(sulfo-3 chloro-4 phényl) (chloro-4 phényl) phosphine ; tri(sulfo-3 phényl)-phosphine ; tri(sulfo-4 phényl)-phosphine ; (tri(sulfo--3 méthyl-4 phényl)-phosphine ; tri(sulfo-3, méthoxy-4 phényl)-phosphine ; tri(sulfo-3 chloro-4 phényl)-phosphine; (sulfo-2 méthyl-4 phényl) (sulfo-3, méthyl-4 phényl) (disulfo-3,5 méthyl-4 phényl)-phosphine ; (sulfo-3 phényl) (sulfo-3 chloro-4 phényl) (disulfo-3,5 chloro-4 phényl)-phosphine.

On peut bien évidemment utiliser un mélange de ces phosphines. En particulier, on peut utiliser un mélange de phosphines mono-, di- et tri-métasulfonées.

Certaines des phosphines hydrosolubles de formule (I) sont disponibles dans le commerce.

Pour la préparation des autres, on peut se référer aux procédés généraux ou particuliers de synthèse des phosphines décrits dans les ouvrages généraux comme HOUBEN-WEYL, Method der organischen Chemie, organische Phosphor Verbindungen, teil 1 (1963).

Enfin, pour la préparation des dérivés hydrosolubles non décrits, on peut à partir des phosphines ne comportant pas de substituants hydrosolubles définis précédemment, procéder à l'introduction d'un ou plusieurs de ces substituants hydrophiles. Ainsi les groupements sulfonates par exemple peuvent être introduits par la réaction de SO₃ dans l'acide sulfurique. Les groupes carboxylates, phosphonates, ammonium quaternaires peuvent de même être introduits en appliquant les méthodes chimiques connues pour ce type de synthèse.

On peut également citer comme composés organophosphorés sulfonés hydrosolubles convenables pour l'invention, les composés BISBIS, NORBOS, BINAS décrits dans l'article de Boy Cornils et Emile G. Kuntz publié dans " Journal of Organometallic Chemistry " n°502 (1995) p177-186. De même les composés furylphosphines hydrosolubles décrits dans la demande de brevet français n° 98 06559 déposée le 20/05/98 non encore publiée rentrent dans le cadre de l'invention.

Comme indiqué précedemment, ces composés organophosphorés désulfités sont notamment uitlisés comme composant de catalyseur comprenant un élément métallique de transition choisi, par exemple, dans le groupe comprenant le nickel, le cobalt, le fer, le ruthénium, le rhodium, le palladium, l'osmium, l'iridium, le platine, le cuivre, l'argent, l'or, le zinc, le cadmium, le mercure à des degrés d'oxydation variables.

A titre d'exemples, on peut indiquer que dans ces catalyseurs, généralement le rhodium est au degré d'oxydation (I), le ruthénium au degré d'oxydation (II), le platine au degré d'oxydation (I), le palladium au degré d'oxydation (II), l'osmium au degré d'oxydation (0), l'iridium au degré d'oxydation (0), le nickel au degré d'oxydation (0).

D'autres détails, avantages de l'invention apparaitront au vu des exemples donnés ci-dessous.

### EXEMPLE 1

Dans un réacteur de 20 litres agité à l'aide d'une turbine (180 tours/minute) et équipé d'un réfrigérant ascendant, d'une arrivée d'azote par canne plongeante et d'un barboteur contenant une solution aqueuse de soude (1 mol/litre), on charge 19,2 kg d'une solution aqueuse à 30% pds du sel de sodium de la triphénylphosphine trisulfonée (TPPTS) et contenant initialement 1540 ppm de sulfites (pH = 6,2). On dégaze cette solution. On introduit ensuite 222 g d'une solution aqueuse d'acide sulfurique à 2 moles/litres, ce qui conduit à un pH de 1,8. On chauffe à 80°C sous agitation et sous courant d'azote de manière à entraîner le gaz sulfureux. Un dosage des sulfites par chromatographie ionique sur des prélèvements effectués toutes les 15 minutes permet de suivre la transformation des sulfites en dioxyde de souffre. Après 1 heure à 80°C, le dosage en question fournit le résultat suivant : 80 ppm de sulfites. Au bout de 1 h 45 minutes, la teneur en sulfites devient inférieure ou égale à 40 ppm (limite de détection de l'analyse par chromatographie ionique). Après 2 heures à 80°C, le mélange est refroidi sous agitation jusqu'à la température ambiante. On introduit alors 324 g d'une solution aqueuse de soude à 1 mole/litre et on obtient une solution aqueuse de TPPTS (pH = 5,4) exempte de sulfites.

### EXEMPLE 2

Dans un ballon en verre de 1 litre, muni d'un barreau aimanté et d'un réfrigérant ascendant, on charge 500 cm³ de la solution aqueuse de TPPTS à 30 % pds exempte de sulfites de l'exemple 1. On dégaze la solution. On introduit ensuite, sous agitation et sous courant d'argon, 20 g de Ni(cyclooctadiène)₂, puis 350 c m³ d'ortho-xylène préalablement dégazé. On chauffe à 45°C pendant 15 h. Après refroidissement, le système biphasique est décanté. Environ 35 cm³ de la phase aqueuse, fortement colorée en rouge, sont prélevés et introduits dans un réacteur en verre de 150 cm³ équipé d'une turbine et purgé à fargon. On chauffe jusqu'à 90°C puis on ajoute 3,2 cm³ d'une solution aqueuse de chlorure de zinc à 70% pds. On maintient le mélange à 90°C sous agitation pendant 48 heures. Après retour à température ambiante, on effectue un prélèvement de la solution aqueuse que l'on analyse par RMN (Résonance Magnétique Nucléaire) du phosphore 31. L'analyse, réalisée sur un spectromètre Bruker AMX 300 ll ® à une fréquence de 121 MHz, montre que le TPPTS ne contient pas de sulfure de TPPTS (teneur inférieure à limite de détection de la technique d'analyse, c'est à dire, inférieure à 0,1 % molaire du phosphore total en solution).

### EXEMPLE 3

Dans un ballon en verre de 1 litre, muni d'un barreau aimanté et d'un réfrigérant ascendant, on charge 500 cm³ de la solution aqueuse de TPPTS à 30 % pds contenant initialement 1540 ppm de sulfites. On dégaze la solution. On introduit ensuite, sous agitation et sous courant d'argon, 20 g de Ni(cyclooctadiène)₂, puis 350 cm³ d'orthoxylène préalablement dégazé. On chauffe à 45°C pendant 15 h. Après refroidissement, le système biphasique est décanté. Environ 35 cm³ de la phase aqueuse, fortement colorée en rouge, sont prélevés et introduits dans un réacteur en verre de 150 cm³ équipé d'une turbine et purgé à l'argon. On chauffe jusqu'à 90°C puis on ajoute 3,2 cm³ d'une solution aqueuse de chlorure de zinc à 70% pds. On maintient le mélange à 90°C sous agitation pendant 48 heures. Après retour à température ambiante, on effectue un prélèvement de la solution aqueuse que l'on analyse par RMN (Résonance Magnétique Nucléaire) du phosphore 31. L'analyse en question, réalisée sur un spectromètre Bruker AMX 300 II ® à une fréquence de 121 MHz, montre qu'il contient du sulfure de TPPTS (pic à 43,7 ppm) à hauteur de 4,5 % molaire du phosphore total en solution.

## Revendications

1. Procédé de mise en oeuvre de réactions organiques par catalyse biphasique comprenant une phase organique contenant les réactifs et les produits de la réaction, une phase aqueuse comprenant un catalyseur et un composé organophosphoré sulfoné hydrosoluble répondant aux formules suivantes : 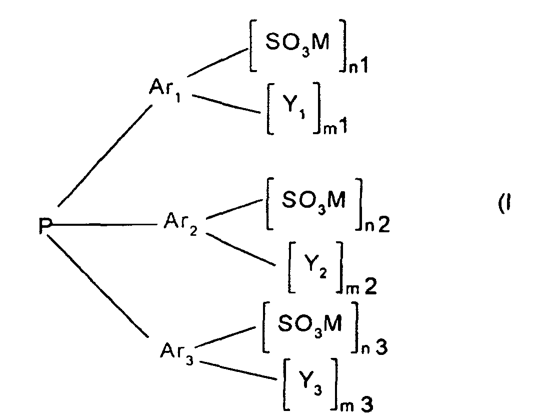 dans laquelle :
* Ar₁, Ar₂ et Ar₃, identiques ou différents, représentent des groupements aryles
* Y₁, Y₂ et Y₃, identiques ou différents, représentent
. un radical alkyle ayant 1 à 4 atomes de carbone,
. un radical alcoxy ayant 1 à 4 atomes de carbone, ,
. un atome d'halogène,
. un radical CN,
. un radical NO₂
. un radical OH,
. un radical NR₁R₂, dans la formule duquel R₁ et R₂, identiques ou différents, représentent un radical alkyle ayant 1 à 4 atomes de carbone,
* M est un reste cationique minéral ou organique choisi, de manière à ce que le composé de formule (I) soit soluble dans l'eau, dans le groupe comprenant :
. H⁺,
. les cations dérivés des métaux alcalins ou alcalino-terreux,
. N(R₃R₄R₅R₆)⁺ avec R₃,R₄,R₅ et R₆, identiques ou différents, représentant un radical alkyle ayant 1 à 4 atomes de carbone ou un atome d'hydrogène,
. les autres cations dérivés des métaux dont les sels de l'acide benzènesulfonique sont solubles dans l'eau,
* m₁, m₂ et m₃ sont des nombres entiers, identiques ou différents, de 0 à 5,
* n₁, n₂ et n₃ sont des nombres entiers, identiques ou différents, de 0 à 3, l'un au moins étant égal ou supérieur à 1,
**caractérisé en ce que** le composé organophosphoré sulfoné hydrosoluble est soumis à un traitement d'élimination au moins partielle des composés ou radicaux sulfites.

2. Procédé selon la revendication 1, **caractérisé en ce que** la concentration pondérale en composés ou radicaux sulfites après traitement est inférieure à 50 ppm.

3. Procédé selon l'une des revendications précédentes, **caractérisé en ce que** la réaction organique est choisi dans le groupe comprenant les réactions d'hydroformylation, carbonylation, d'oxydation, d'isomérisation, d'hydrocyanation.

4. Procédé selon la revendication 3, **caractérisé en ce que** la réaction organique est une hydrocyanation de composés organiques comprenant au moins une insaturation éthylénique.

5. Procédé selon la revendication 4, **caractérisé en ce que** le composé organique est une dioléfine, ou du 3-pentènenitrile.

6. Procédé selon l'une des revendications précédentes, **caractérisé en ce que** le catalyseur comprend le composé organophosphoré sulfoné hydrosoluble et au moins un métal de transition.

7. Procédé selon la revendication 6, **caractérisé en ce que** le métal de transition est choisi dans le groupe comprenant le nickel, le cobalt, le fer, le ruthénium, le rhodium, le palladium, l'osmium, l'iridium, le platine, le cuivre, l'argent, l'or, le zinc, le cadmium, le mercure.

8. Procédé selon la revendication 6 ou 7, **caractérisé en ce que** le catalyseur comprend des éléments métalliques promoteurs ou dopants.

9. Procédé de préparation de composés organophosphorés sulfonés hydrosolubles correspondants aux formules générales suivantes : 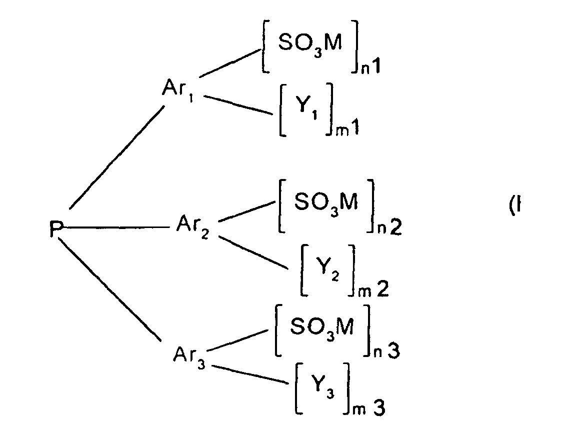 dans laquelle :
* Ar₁, Ar₂ et Ar₃, identiques ou différents, représentent des groupements aryles
* Y₁, Y₂ et Y₃, identiques ou différents, représentent
. un radical alkyle ayant 1 à 4 atomes de carbone,
. un radical alcoxy ayant 1 à 4 atomes de carbone,
. un atome d'halogène,
. un radical CN,
. un radical NO₂
. un radical OH,
. un radical NR₁ R₂, dans la formule duquel R₁ et R₂, identiques ou différents, représentent un radical alkyle ayant 1 à 4 atomes de carbone,
* M est un reste cationique minéral ou organique choisi, de manière à ce que le composé de formule (1) soit soluble dans l'eau, dans le groupe comprenant:
. H⁺
. les cations dérivés des métaux alcalins ou alcalino-terreux,
. N(R₃R₄R₅R₆)⁺ avec R₃,R₄,R₅ et R₆, identiques ou différents, représentant un radical alkyle ayant 1 à 4 atomes de carbone ou un atome d'hydrogène,
. les autres cations dérivés des métaux dont les sels de l'acide benzènesulfonique sont solubles dans l'eau,
* m₁, m₂ et m₃ sont des nombres entiers, identiques ou différents, de 0 à 5,
* n₁, n₂ et n₃ sont des nombres entiers, identiques ou différents, de 0 à 3, l'un au moins étant égal ou supérieur à 1,
**caractérisé en ce qu'**il consiste à éliminer au moins partiellement les composés ou groupes sulfites par abaissement du pH d'une solution desdits composés à une valeur inférieure ou égale à 4, et maintient du pH de cette solution à une valeur inférieure ou égale à 4 jusqu'à une concentration pondérale en sulfite dans la solution à une valeur inférieure à 100 ppm.

10. Procédé selon la revendication 9, **caractérisé en ce que** l'abaissement du pH est réalisé par addition d'un composé acide.

11. Procédé selon la revendication 10, **caractérisé en ce que** l'acide est un acide fort minéral ou organique.

12. Procédé selon la revendication 11, **caractérisé en ce que** l'acide a un pKa inférieur ou égal à 4.

13. Procédé selon l'une des revendications 10 à 12, **caractérisé en ce que** le composé acide est choisi dans le groupe comprenant l'acide sulfurique, l'acide chlorhydrique, l'acide trifluoroacétique, l'acide paratoluène sulfonique, l'acide perchlorique, l'acide nitrique.

14. Procédé selon l'une des revendications 9 à 13 **caractérisé en ce que** la solution est maintenue à une température inférieure à 100°C, de préférence comprise entre 40 et 90°C.

15. Procédé selon l'une des revendications 9 à 14, **caractérisé en ce que** le gaz sulfureux éliminé par entraînement par un fluide vecteur.

16. Procédé selon la revendication 15, **caractérisé en ce que** le fluide vecteur est choisi dans le groupe comprenant l'azote, le gaz carbonique, la vapeur d'eau, les gaz rares ou inertes, de l'air appauvri en oxygène.

17. Procédé selon l'une des revendications 9 à 16, **caractérisé en ce que** la solution dudit composé organophosphoré sulfoné est une solution aqueuse ou une solution dans un mélange eau/alcool.

## Claims

1. Process for carrying out organic reactions by two-phase catalysis comprising an organic phase comprising the reactants and the reaction products and an aqueous phase comprising a catalyst and a water-soluble sulphonated organophosphorus compound corresponding to the following formula: 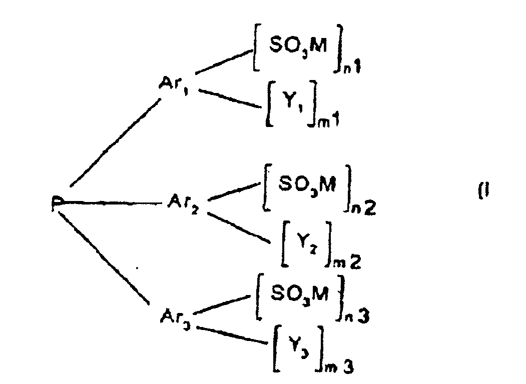 in which:
• Ar₁, Ar₂ and Ar₃, which are identical or different, represent aryl groups,
• Y₁, Y₂ and Y₃, which are identical or different, represent
• an alkyl radical having 1 to 4 carbon atoms,
• an alkoxy radical having 1 to 4 carbon atoms,
• a halogen atom,
• a CN radical,
• an NO₂ radical,
• an OH radical,
• an NR₁R₂ radical, in the formula of which R₁ and R₂, which are identical or different, represent an alkyl radical having 1 to 4 carbon atoms,
• M is an inorganic or organic cationic residue chosen, so that the compound of formula (I) is soluble in water, from the group consisting of:
• H⁺,
• cations derived from alkali metals or alkaline earth metals,
• N (R₃R₄R₅R₆)⁺, with R₃, R₄, R₅ and R₆, which are identical or different, representing an alkyl radical having 1 to 4 carbon atoms or a hydrogen atom,
• other cations derived from metals, the benzenesulphonic acid salts of which are soluble in water,
• m₁, m₂ and m₃ are identical or different integers from 0 to 5,
• n₁, n₂ and n₃ are identical or different integers from 0 to 3, one at least being equal or greater than 1,
**characterized in that** the water-soluble sulphonated organophosphorus compound is subjected to a treatment for at least partial removal of the sulphite compounds or radicals.

2. Process according to Claim 1, **characterized in that** the concentration by weight of sulphite compounds or radicals after treatment is less than 50 ppm.

3. Process according to either of the preceding claims, **characterized in that** the organic reaction is chosen from the group consisting of the hydroformylation, carbonylation, oxidation, isomerization and hydrocyanation reactions.

4. Process according to Claim 3, **characterized in that** the organic reaction is a hydrocyanation of organic compounds comprising at least one ethylenic unsaturation.

5. Process according to Claim 4, **characterized in that** the organic compound is a diolefin or 3-pentenenitrile.

6. Process according to one of the preceding claims, **characterized in that** the catalyst comprises the water-soluble sulphonated organophosphorus compound and at least one transition metal.

7. Process according to Claim 6, **characterized in that** the transition metal is chosen from the group consisting of nickel, cobalt, iron, ruthenium, rhodium, palladium, osmium, iridium, platinum, copper, silver, gold, zinc, cadmium and mercury.

8. Process according to Claim 6 or 7, **characterized in that** the catalyst comprises promoting or doping metal elements.

9. Process for the preparation of water-soluble sulphonated organophosphorus compounds corresponding to the following general formula: 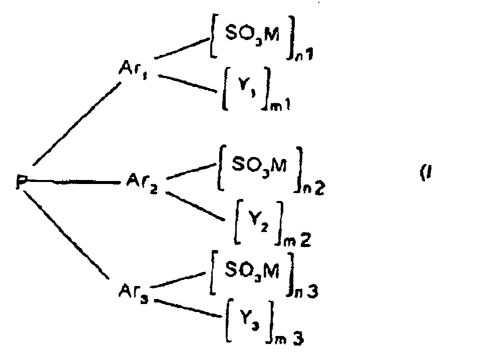 in which:
• Ar₁, Ar₂ and Ar₃, which are identical or different, represent aryl groups,
• Y₁, Y₂ and Y₃, which are identical or different, represent
• an alkyl radical having 1 to 4 carbon atoms,
• an alkoxy radical having 1 to 4 carbon atoms,
• a halogen atom,
• a CN radical,
• an NO₂ radical,
• an OH radical,
• an NR₁R₂ radical, in the formula of which R₁ and R₂, which are identical or different, represent an alkyl radical having 1 to 4 carbon atoms,
• M is an inorganic or organic cationic residue chosen, so that the compound of formula (I) is soluble in water, from the group consisting of:
• H⁺,
• cations derived from alkali metals or alkaline earth metals,
• N (R₃R₄R₅R₆)⁺, with R₃, R₄, R₅ and R₆, which are identical or different, representing an alkyl radical having 1 to 4 carbon atoms or a hydrogen atom,
• other cations derived from metals, the benzenesulphonic acid salts of which are soluble in water,
• m₁, m₂ and m₃ are identical or different integers from 0 to 5,
• n₁, n₂ and n₃ are identical or different integers from 0 to 3, one at least being equal or greater than 1
**characterized in that** it consists in at least partially removing the sulphite compounds or groups by lowering the pH of a solution of the said compounds to a value of less than or equal to 4 and by maintaining the pH of this solution at a value of less than or equal to 4 until a concentration by weight of sulphite in the solution has a value of less than 100 ppm.

10. Process according to Claim 9, **characterized in that** the pH is lowered by addition of an acidic compound.

11. Process according to Claim 10, **characterized in that** the acid is a strong inorganic or organic acid.

12. Process according to Claim 11, **characterized in that** the acid has a pKa of less than or equal to 4.

13. Process according to one of Claims 10 to 12, **characterized in that** the acidic compound is chosen from the group consisting of sulphuric acid, hydrochloric acid, trifluoracetic acid, para-toluenesulphonic acid, perchloric acid and nitric acid.

14. Process according to one of Claims 9 to 13, **characterized in that** the solution is maintained at a temperature of less than 100°C, preferably of between 40 and 90°C.

15. Process according to one of Claims 9 to 14, **characterized in that** the sulphur dioxide gas is removed by entrainment with a carrier fluid.

16. Process according to Claim 15, **characterized in that** the carrier fluid is chosen from the group consisting of nitrogen, carbon dioxide, steam, rare or inert gases, and air depleted in oxygen.

17. Process according to one of Claims 9 to 16, **characterized in that** the solution of the said sulphonated organophosphorus compound is an aqueous solution or a solution in a water/alcohol mixture.

## Patentansprüche

1. Verfahren zur Durchführung von organischen Reaktionen durch biphasische Katalyse, umfassend eine organische Phase, welche die Reaktanden und die Produkte der Reaktion enthält, und eine wäßrige Phase die einen Katalysator und eine wasserlösliche sulfonierte Organophosphor-Verbindung umfaßt, die den folgenden Formeln entspricht: 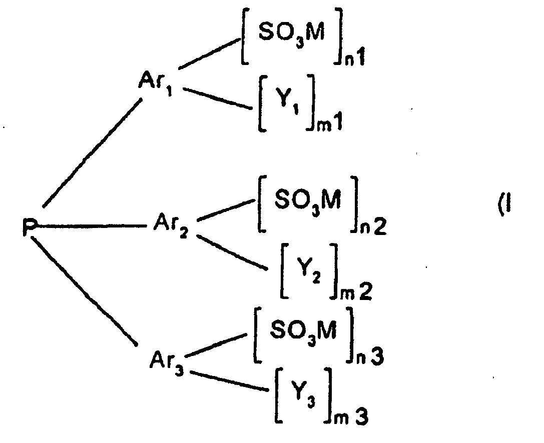 in der
* Ar₁, Ar₂ und Ar₃, gleich oder verschieden, Arylgruppen darstellen,
* Y₁, Y₂ und Y₃, gleich oder verschieden, darstellen
• einen Rest Alkyl mit 1 bis 4 Kohlenstoffatomen,
• einen Rest Alkoxy mit 1 bis 4 Kohlenstoffatomen,
• ein Halogenatom,
• einen Rest CN,
• einen Rest NO₂,
• einen Rest OH,
• einen Rest NR₁R₂, worin R₁ und R₂, gleich oder verschieden, einen Rest Alkyl mit 1 bis 4 Kohlenstoffatomen darstellen.
* M einen mineralischen oder organischen kationischen Rest in der Weise darstellt, daß die Verbindung der Formel (I) in Wasser löslich ist, in der Gruppe umfassend:
· H⁺,
· die Kationen, abgeleitet von Alkalimetallen oder Erdalkalimetallen,
· N(R₃R₄R₅R₆)⁺ mit R₃, R₄, R₅ und R₆, gleich oder verschieden, die einen Rest Alkyl mit 1 bis 4 Kohlenstoffatomen oder ein Wasserstoffatom darstellen,
· die anderen Ionen, abgeleitet von Metallen, deren Salze der Benzolsulfonsäure in Wasser löslich sind,
* m₁, m₂ und m₃, gleich oder verschieden, ganze Zahlen von 0 bis 5 sind,
* n₁, n₂ und n₃, gleich oder verschieden, ganze Zahlen von 0 bis 3 sind, wobei mindestens eine gleich oder über 1 beträgt,
**dadurch gekennzeichnet, daß** die wasserlösliche sulfonierte Organophosphor-Verbindung einer Behandlung zur mindestens teilweisen Entfernung der Sulfit-Verbindungen oder Sulfit-Reste unterzogen wird.

2. Verfahren nach Anspruch 1, **dadurch gekennzeichnet, daß** die gewichtsmäßige Konzentration an Sulfit-Verbindungen oder Sulfit-Resten nach der Behandlung unter 50 ppm beträgt.

3. Verfahren nach einem der vorstehenden Ansprüche, **dadurch gekennzeichnet, daß** die organische Reaktion aus der Gruppe gewählt wird, welche die Reaktionen der Hydroformylierung, Carbonylierung, Oxidation, Isomerisierung und Hydrocyanierung umfaßt.

4. Verfahren nach Anspruch 3, **dadurch gekennzeichnet, daß** die organische Reaktion eine Hydrocyanierung von organischen Verbindungen ist, die mindestens eine ethylenische Unsättigung umfassen.

5. Verfahren nach Anspruch 4, **dadurch gekennzeichnet, daß** die organische Verbindung ein Diolefin oder 3-Pentennitril ist.

6. Verfahren nach einem der vorstehenden Ansprüche, **dadurch gekennzeichnet, daß** der Katalysator die wasserlösliche sulfonierte Organophosphor-Verbindung und mindestens ein Übergangsmetall umfaßt.

7. Verfahren nach Anspruch 6, **dadurch gekennzeichnet, daß** das Übergangsmetall aus der Gruppe gewählt wird, die Nickel, Cobalt, Eisen, Ruthenium, Rhodium, Palladium, Osmium, Iridium, Platin, Kupfer, Silber, Gold, Zink, Cadmium, Quecksilber umfaßt.

8. Verfahren nach Anspruch 6 oder 7, **dadurch gekennzeichnet, daß** der Katalysator metallische Promotor-Elemente oder Dotierungsmittel umfaßt.

9. Verfahren zur Herstellung der wasserlöslichen sulfonierten Organophosphor-Verbindungen, die den folgenden allgemeinen Formeln entsprechen: 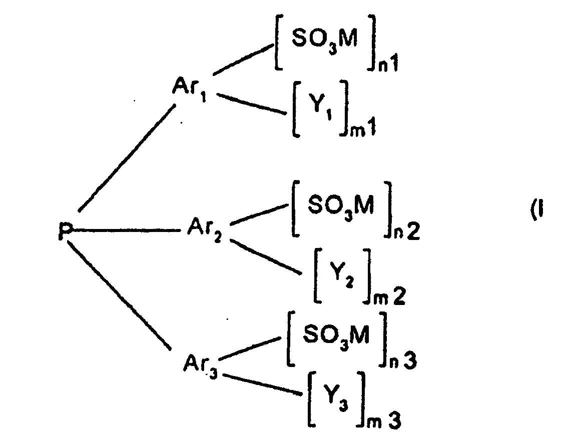 in der
* Ar₁, Ar₂ und Ar₃, gleich oder verschieden, Arylgruppen darstellen,
* Y₁, Y₂ und Y₃, gleich oder verschieden, darstellen
· einen Rest Alkyl mit 1 bis 4 Kohlenstoffatomen,
· einen Rest Alkoxy mit 1 bis 4 Kohlenstoffatomen,
· ein Halogenatom,
· einen Rest CN,
· einen Rest NO₂,
· einen Rest OH,
· einen Rest NR₁R₂, worin R₁ und R₂, gleich oder verschieden, einen Rest Alkyl mit 1 bis 4 Kohlenstoffatomen darstellen.
* M einen mineralischen oder organischen kationischen Rest in der Weise darstellt, daß die Verbindung der Formel (I) in Wasser löslich ist, in der Gruppe umfassend:
· H⁺,
· die Kationen, abgeleitet von Alkalimetallen oder Erdalkalimetallen,
• N(R₃R₄R₅R₆)⁺ mit R₃, R₄, R₅ und R₆, gleich oder verschieden, die einen Rest Alkyl mit 1 bis 4 Kohlenstoffatomen oder ein Wasserstoffatom darstellen,
• die anderen Ionen, abgeleitet von Metallen, deren Salze der Benzolsulfonsäure in Wasser löslich sind,
* m₁, m₂ und m₃, gleich oder verschieden, ganze Zahlen von 0 bis 5 sind,
* n₁, n₂ und n₃, gleich oder verschieden, ganze Zahlen von 0 bis 3 sind, wobei mindestens eine gleich oder über 1 beträgt,
**dadurch gekennzeichnet, daß** es darin besteht, mindestens teilweise die Sulfit-Verbindungen oder Sulfit-Gruppen zu entfernen durch Absenkung des pH-Wertes einer Lösung der genannten Verbindungen auf einen Wert von unter oder gleich 4 und Aufrechterhalten des pH-Wertes von dieser Lösung auf einem Wert von unter oder gleich 4 bis zu einer gewichtsmäßigen Konzentration an Sulfit in der Lösung von einen Wert unter 100 ppm.

10. Verfahren nach Anspruch 9, **dadurch gekennzeichnet, daß** die Absenkung des pH-Wertes durch Zugabe einer sauren Verbindung realisiert wird.

11. Verfahren nach Anspruch 10, **dadurch gekennzeichnet, daß** die Säure eine starke Mineralsäure oder organische Säure ist.

12. Verfahren nach Anspruch 11, **dadurch gekennzeichnet, daß** die Säure einen pKa von unter oder gleich 4 besitzt.

13. Verfahren nach einem der Ansprüche 10 bis 12, **dadurch gekennzeichnet, daß** die saure Verbindung aus der Gruppe gewählt wird, die Schwefelsäure, Chlorwasserstoffsäure, Trifluoressigsäure, para-Toluolsulfonsäure, Perchlorsäure, Salpetersäure umfaßt.

14. Verfahren nach einem der Ansprüche 9 bis 13, **dadurch gekennzeichnet, daß** die Lösung auf einer Temperatur von unter 100 °C, vorzugsweise zwischen einschließlich 40 °C und 90 °C gehalten wird.

15. Verfahren nach einem der Ansprüche 9 bis 14, **dadurch gekennzeichnet, daß** das Schwefeldioxid durch Mitnahme mit einem fluiden Trägermittel entfernt wird.

16. Verfahren nach Anspruch 15, **dadurch gekennzeichnet, daß** das fluide Trägermittel aus der Gruppe gewählt wird, die Stickstoff, Kohlendioxid, Wasserdampf, seltene oder inerte Gase, an Sauerstoff abgereicherte Luft umfaßt.

17. Verfahren nach einem der Ansprüche 9 bis 16, **dadurch gekennzeichnet, daß** die Lösung der genannten sulfonierten Organophosphor-Verbindung eine wäßrige Lösung oder eine Lösung in einer Mischung Wasser/Alkohol ist.
